# EUROPÄISCHE PATENTANMELDUNG

(11) **EP 3 903 834 A2**
(43) Veröffentlichungstag der Anmeldung: **03.11.2021**
(21) Anmeldenummer: 21166033.7
(22) Anmeldetag: 30.03.2021
(51) Int. Cl.: A61L 2/06

(54) **VERFAHREN ZUM DESINFIZIEREN VON BIOLOGISCH KONTAMINIERTEN GEGENSTÄNDEN**

(30) Priorität: 06.04.2020 DE 102020002171
(71) Anmelder: Herbert Kannegiesser GmbH, 32602 Vlotho (DE)
(72) Erfinder: HEINZ Engelbert, 32602 Vlotho (DE); BRINGEWATT Wilhelm, 32457 Porta Westfalica (DE)
(74) Vertreter: Möller, Friedrich

(57) **Zusammenfassung**

Biologisch kontaminierte Gegenstände müssen desinfiziert werden, damit sie nicht mehr infektiös und erneut einsetzbar sind. Es ist bekannt, Wäschestücke während des Waschvorgangs chemisch zu desinfizieren. Diese Art der Desinfektion eignet sich aber nicht für alle kontaminierten Gegenstände.

Die Erfindung sieht es vor, kontaminierte Gegenstände im trockenen Zustand thermisch zu desinfizieren durch Beaufschlagung mit heißer Luft. Eine solche thermische Desinfektion kann in Trocknern für gewaschene Wäsche erfolgen.

## Beschreibung

Die Erfindung betrifft ein Verfahren zum Desinfizieren von biologisch kontaminierten Gegenstände, vorzugsweise biologisch kontaminierten textilen Gegenständen.

Kontaminierte Gegenstände bzw. mikrobiell-belastete Gegenstände müssen vor einer erneuten Verwendung zuverlässig desinfiziert werden. Bei den Gegenständen kann es sich um textile Gegenstände, beispielsweise Wäsche aus Krankenhäusern, oder auch mikrobiell-belastete Schutzgegenstände aus sowohl textilen als auch nicht-textilen Materialien, beispielsweise Kunststoff, handeln. Solche Schutzgegenstände betreffen vor allem Gesichtsmasken, Mundschutz aus textilem Material, Handschuhe, Fußschutz und textile oder auch nicht-textile Schutzkleidung

Solche Gegenstände werden vor erneutem Gebrauch nicht gewaschen, weil sie nicht gewaschen werden müssen oder können. Demzufolge werden solche Gegenstände lediglich von ihrer biologischen bzw. mikrobiellen Kontamination, beispielsweise Keimen, Viren, Pilzen und/oder Bakterien befreit. Damit wird sichergestellt, dass die Desinfektion die Kontamination vollständig beseitigt, damit sie für nachfolgende Benutzer nicht mehr infektiös ist.

Ausgehend vom Vorstehenden liegt der Erfindung zugrunde, ein Verfahren zur einfachen, aber gleichwohl zuverlässigen vollständigen Desinfektion von biologisch bzw. mikrobiell kontaminierten Gegenständen zu schaffen.

Ein Verfahren zur Lösung der Aufgabe weist die Maßnahmen des Anspruchs 1 auf. Demnach werden die biologisch kontaminierten Gegenstände im trockenen Zustand mit Heißluft beaufschlagt. Trocken bedeutet, dass die Gegenstände noch eine geringe Restfeuchte aufweisen können, die so gering ist, dass die Gegenstände gebrauchs- und lagerfähig sind (schranktrocken). Durch die Behandlung mit Heißluft werden die Kontaminationen auf einfache Weise wirksam beseitigt. Vor allem werden Bakterien, Keime, Viren und andere mikrobielle Verunreinigungen bzw. Kontaminationen der Gegenstände beseitigt. Das führt dazu, dass die trocken desinfizierten Gegenstände mindestens größtenteils steril sind.

Bevorzugt ist es vorgesehen, die biologisch kontaminierten Gegenstände, insbesondere biologisch kontaminierten textilen Gegenstände, mit einer Vorrichtung nach Art eines Trockners mit Heißluft zu desinfizieren. Es findet so eine nur thermische Desinfektion der kontaminierten Gegenstände statt. Diese kann im trockenen Zustand erfolgen, ohne dass es erforderlich ist, die kontaminierten Gegenstände auf andere Weise, insbesondere chemisch und/oder mechanisch, zu desinfizieren und/oder zu waschen.

Gemäß einer bevorzugten Ausgestaltung des Verfahrens ist es vorgesehen, dass die zu desinfizierenden Gegenstände von der Heißluft umströmt werden. Das kann vorzugsweise durch eine Umwälzung der Heißluft geschehen, wobei gegebenenfalls die Desinfektion der Gegenstände wenigstens teilweise, insbesondere größtenteils oder vollständig, mit heißer Umluft erfolgt.

Besonders vorteilhaft ist es, wenn die Heißluft auf eine Desinfektions-Temperatur aufgeheizt wird, die 20% bis 50% unter derjenigen Temperatur liegt, womit die jeweiligen zu desinfizierenden Gegenstände maximal beschädigungslos beaufschlagbar sind. Beispielsweise ist es diejenige Temperatur, bei der Textilien noch nicht thermisch überbeansprucht werden und im Extremfall versengen.

Eine Möglichkeit der Weiterbildung des Verfahrens besteht darin, dass zur sicheren Abtötung aller oder zumindest nahezu aller biologischen bzw. mikrobiellen Kontaminationen der zu desinfizierenden Gegenstände die Desinfektions-Temperatur im Bereich von 70°C bis 98°C, vorzugsweise 85°C bis 95°C, liegt. Solche Temperaturen sind von Gegenständen, die aufgrund ihres Einsatzes häufiger desinfiziert werden müssen, typischerweise unschädlich, aber hoch genug, um mikrobielle oder biologische Kontaminationen, insbesondere Bakterien, Viren, Keime, Pilze oder dergleichen sicher abzutöten, so dass das erfindungsgemäße Verfahren es ermöglicht, zumindest einen Großteil der Gegenstände bei der Desinfektion so weit von Bakterien, Viren, Keimen oder sonstigen mikrobiellen Kontaminationen zu befreien, dass sie als steril gelten.

Das Verfahren ist bevorzugt so ausgestaltet, dass die zu desinfizierenden Gegenstände über einen bestimmten und/oder festgelegten, ununterbrochenen Zeitraum hinweg mit der die Desinfektions-Temperatur mindestens aufweisenden Heißluft beaufschlagt werden. Bevorzugt beträgt dieser Zeitraum 5 min bis 15 min, insbesondere 8 min bis 12 min. Innerhalb dieses Zeitraums werden die zu desinfizierenden kontaminierten Gegenstände durchgehend mit Heißluft beaufschlagt, die mindestens die Desinfektions-Temperatur aufweist, vorzugsweise diese nicht oder zumindest nicht nennenswert überschreitet, damit bei der Desinfektion über diesen Zeitraum hinweg die zu desinfizierenden Gegenstände, insbesondere wenn es sich dabei um textile Gegenstände handelt, nicht thermisch überbeansprucht werden und dadurch Schaden nehmen könnten.

Eine vorteilhafte Ausgestaltungsmöglichkeit des Verfahrens sieht es vor, dass die Desinfektions-Temperatur während des Desinfektionsvorgangs der kontaminierten Gegenstände mit der Heißluft mit mindestens einem Messorgan, vorzugsweise mindestens zwei Messorganen, gemessen wird. Diese Messung erfolgt insbesondere fortlaufend oder in regelmäßigen Zeitabständen. Hierdurch wird der Desinfektionsvorgang dahingehend überwacht, ob während des gesamten Zeitraums, den die Desinfektion erfolgen muss, die Desinfektions-Temperatur im vorgegebenen oder vorgesehenen Bereich liegt. Werden bei dieser Überprüfung Abweichungen festgestellt, indem die Desinfektions-Temperatur unterschritten wird, dann wird die Zeitdauer der Desinfektion um einen solchen Zeitraum verlängert, in dem während der Desinfektion die Desinfektions-Temperatur unter dem vorgegebenen Wert lag. Bei überhöhter Desinfektions-Temperatur wird lediglich die Temperatur der Heißluft möglichst rasch auf den vorgesehenen Wert reduziert, damit es bei der Desinfektion zu keinen Beschädigungen der zu desinfizierenden kontaminierten Gegenstände, insbesondere textiler Gegenständel, kommt, aber die Desinfektionsdauer nicht reduziert.

Das mindestens eine Messorgan zur Überwachung der Desinfektions-Temperatur ist bevorzugt so ausgebildet, dass es die Oberflächentemperatur der zu desinfizierenden Gegenstände berührungslos misst. Diese Messung kann während des laufenden Desinfektionsvorgangs erfolgen, insbesondere bei zum Beispiel von der drehend antreibbaren Trommel bewegten Gegenständen.

Vorzugsweise sind zwei oder auch mehr als zwei Messorgane vorgesehen. Dabei kann es sich um gleiche Messorgane handeln. Dadurch wird eine Redundanz geschaffen und verhindert, dass beim Ausfall eines Messorgans die Temperaturüberwachung während eines laufenden Desinfektionsvorgangs unterbrochen wird.

Eine andere vorteilhafte Ausgestaltungsmöglichkeit des Verfahrens sieht es vor, dass nass zu behandelnde Gegenstände, insbesondere textile Gegenstände, vor einer Nassbehandlung im trockenen Zustand durch Beaufschlagung mit Heißluft desinfiziert werden. Dadurch können die zuvor desinfizierten Gegenstände, insbesondere kontaminierte Wäsche, im Anschluss an die der Nassbehandlung vorausgegangenen Desinfektion weiteren Behandlungen, insbesondere der Nassbehandlung, genauso unterzogen werden wie nicht biologisch kontaminierte Gegenstände, insbesondere Wäsche, die nicht infektiös ist.

Das Verfahren kann so ausgebildet sein, dass die kontaminierten Gegenstände postenweise, vorzugsweise als Posten sortierter Gegenstände, sortenrein desinfiziert werden. Dann wird vorzugsweise eine solche Desinfektion in einer drehend antreibbaren und für die Heißluft durchlässige und/oder durchströmbare Trommel vorgenommen. Die Heißluft umströmt dabei die zu desinfizierenden Gegenstände in der Trommel und beseitigt biologische Kontaminationen. Dabei werden Keime, Viren, Pilze und dergleichen zumindest größtenteils vollständig abgetötet und dadurch die Gegenstände mit Heißluft zumindest nahezu sterilisiert.

Es ist auch möglich, das Verfahren zur Desinfektion kontaminierter Gegenstände, insbesondere textiler Gegenstände, nach dem Durchlaufprinzip durchzuführen, indem die zu desinfizierenden Gegenstände durch eine Art Tunnel transportiert werden, und zwar vorzugsweise kontinuierlich. In dem Tunnel wirkt die Heißluft auf die zu desinfizierenden Gegenstände ein, sodass sie vollständig dekontaminiert werden, vorzugsweise soweit, dass sie steril sind. Das Desinfizieren der Gegenstände im Durchlauf durch einen Tunnel kann kontinuierlich erfolgen und eignet sich insbesondere für empfindliche Gegenstände, die in keiner Trommel "umhergeschleudert" werden dürfen.

Bevorzugt ist es erfindungsgemäß vorgesehen, als Heißluft trockene, aufgeheizte Luft zu verwenden. Solche aufgeheizte Luft ist auf vielfältige Weise mit einfachen Lufterhitzern erzeugbar. Im einfachsten Falle kann die zur Desinfektion erforderliche Heißluft mit einer Temperatur von 70°C bis 98°C durch elektrisch arbeitende Heizeinrichtungen erzeugt werden. Bei der Luft kann es sich um trockene Luft handeln. Das kann trockene Umgebungsluft sein, die dann, wenn sie noch zu viel Feuchtigkeit aufweist, beim Aufheizen getrocknet wird. Alternativ kann es auch vorgesehen sein, getrocknete Luft zu verwenden, die dann durch nachträgliches Aufheizen auf die Desinfektions-Temperatur als Heißluft zum Desinfizieren der biologisch und/oder mikrobiell kontaminierten Gegenstände, insbesondere textile Gegenstände, verwendet wird.

In den Figuren der Zeichnung sind zwei mögliche Vorrichtungen zur Durchführung des erfindungsgemäßen Verfahrens, nämlich Desinfektionen kontaminierter Gegenstände, beschrieben. In dieser zeigen:
- Fig. 1: eine schematische Darstellung einer Vorrichtung nach dem Prinzip eines Trommeltrockners, und
- Fig. 2: eine schematische Darstellung einer Vorrichtung nach dem Prinzip eines Durchlauftrockners.

Das erfindungsgemäße Verfahren eignet sich besonders zur Desinfektion kontaminierter, insbesondere mikrobiell kontaminierter, Gegenstände. Die Gegenstände können mit Keimen, Bakterien, Viren, Pilzen und Ähnlichem mikrobiell kontaminiert und dadurch insbesondere infektiös sein. Bei den Gegenständen handelt es sich um benutzte textile Gegenstände, aber auch Gegenstände aus nicht textilen Materialien, beispielsweise Kunststoff. Solche Gegenstände sind insbesondere von medizinischem und klinischen Personal sowie Patienten benutzte infektiöse Gegenstände, wie Schutzgegenstände, zum Beispiel Gesichtsmasken, Atemschutzmasken, Schutzbrillen und Schutzkleidung.

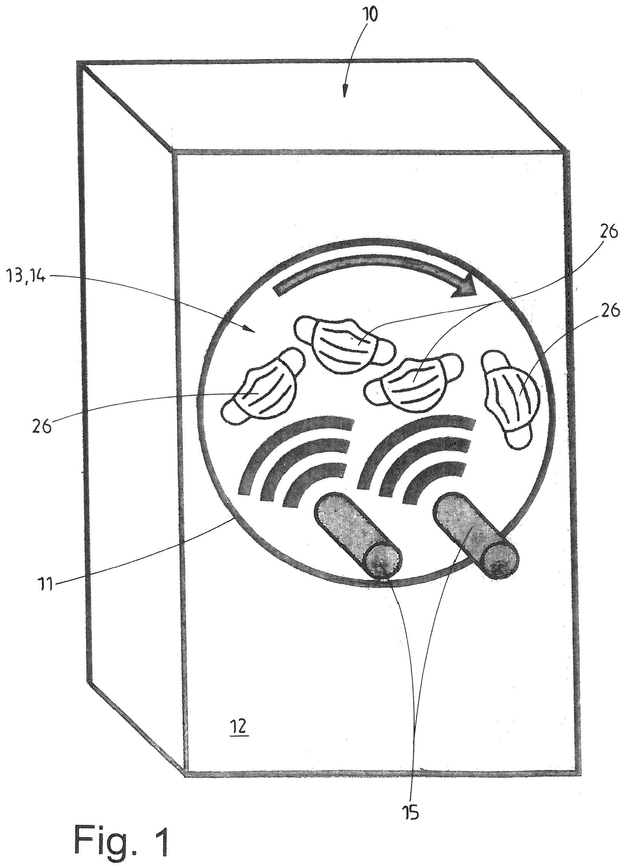

Die Fig. 1 veranschaulicht schematisch eine nach Art eines Trommeltrockners ausgebildete Vorrichtung. Diese Vorrichtung wird für das erfindungsgemäße Verfahren, aber nicht auch zum Trocknen der zu desinfizierenden Gegenstände, nämlich in der Figur beispielhaft dargestellte Schutzmasken 26, eingesetzt, sondern nur zum Desinfizieren derselben durch Aufheizen.

Die trocknerartige Vorrichtung verfügt über ein Gehäuse 10, das vorzugsweise als luftdichtes Gehäuse 10 ausgebildet ist. Im Inneren des Gehäuses 10 befindet sich eine im gezeigten Ausführungsbeispiel um eine horizontale Drehachse drehbare Trommel 11.

Diese Trommel 11 ist bevorzugt als zylindrische Trommel 11 ausgebildet. Die Trommel 11 ist luftdurchlässig ausgebildet. Dazu ist zumindest der zylindrische Mantel der Trommel 11 perforiert, siebartig oder netzartig ausgebildet. Dadurch kann die die zu desinfizierenden Gegenstände aufnehmende Trommel 11 von Heißluft durchströmt werden, die auch die zu desinfizierenden Gegenstände in der Trommel 11 umströmt.

Bei der hier gezeigten Vorrichtung ist eine Vorderwand 12 des Gehäuses 10 mit einer Öffnung 13 versehen. Ebenso weist die hinter der Öffnung 13 liegende Stirnseite der Trommel 11 eine Öffnung auf. Zumindest die Öffnung 13 in der Vorderwand 12 des Gehäuses 10 ist mit einer zu öffnenden Tür 14 oder Klappe verschließbar. Die Tür 14 bzw. Klappe können durchsichtig ausgebildet sein, indem sie beispielsweise aus Glas bestehen. Alternativ oder zusätzlich kann auch die Öffnung in der Stirnseite der Trommel 11 durch eine zu öffnende Tür oder Klappe verschließbar sein. Denkbar ist es auch, dass das Gehäuse 10 und/oder die Trommel 11 an der Rückseite über mindestens eine Öffnung oder Tür verfügen. In diesem Falle erfolgt die Beladung der Trommel mit der zu desinfizierenden Gegenstände bei geöffneter Tür 14 durch die Öffnung 13 in der Vorderwand 12 des Gehäuses 10. Die Entladung hingegen erfolgt dann durch die geöffnete Tür oder Klappe in der Rückwand des Gehäuses 10. Dann ist die Vorrichtung aus einem unreinen Raum mit kontaminierten Gegenständen beladbar, während das Entladen desinfizierter Gegenstände über die rückseitige Öffnung in einen Reinraum erfolgt.

In das Gehäuse 10 ist aufgeheizte Luft, nämlich Heißluft, einblasbar. Die Trommel 11 mit den zu desinfizierenden Gegenständen durch- und/oder umströmte Heißluft ist vorzugsweise aus dem Gehäuse 10 herausführbar. Die aus der Trommel 11 und dem Gehäuse 10 herausgeführte Heißluft kann durch mindestens eine Umlaufleitung zurückgeführt werden zu einer Luftzufuhröffnung im Gehäuse 10. Dadurch ist die Heißluft im Umluftbetrieb durch mindestens einen Ventilator umwälzbar. Dann erfolgt die Desinfektion der kontaminierten Gegenstände im Umluftbetrieb. Es ist auch denkbar, die Heißluft bei Bedarf ganz oder teilweise durch frische aufgeheizte Luft zu ersetzen, sodass ein Mischbetrieb mit Umluft und frischer Heißluft erfolgt.

Die Luft wird erhitzt durch mindestens eine Heizeinrichtung, die der Vorrichtung zugeordnet ist, aber auch außerhalb und entfernt von der Vorrichtung angeordnet sein kann. Die Heißluft kann dadurch zentral und/oder dezentral erzeugt werden. Die Luft kann durch eine elektrisch oder auch mit fossilen Brennstoffen betriebene Heizeinrichtung aufgeheizt werden.

Die mindestens eine Heizeinrichtung ist mit wenigstens einer Temperaturmesseinrichtung versehen, die die Temperatur, vorzugsweise Desinfektions-Temperatur, der Heißluft überwacht. Auch kann die die Trommel verlassende benutzte Heißluft hinsichtlich ihrer Temperatur durch geeignete Temperaturmesseinrichtungen ermittelt werden. Die Ermittlung der Temperaturen geschieht vorzugsweise kontinuierlich. Dadurch ist stets bekannt, welche Temperatur die der Trommel 11 zugeführte Heißluft und gegebenenfalls die Trommel verlassende benutzte Heißluft aufweisen. Durch eine entsprechende Steuereinrichtung kann die Heizeinrichtung so gesteuert oder geregelt werden, dass sie die der Trommel 11 zugeführte Heißluft auf die gewünschte und/oder notwendige Desinfektions-Temperatur aufheizt.

Der hier gezeigten Vorrichtung sind zwei gleiche Sensoren, vorzugsweise Infrarotsensoren 15, zugeordnet. Durch die Infrarotsensoren 15 ist berührungslos die Temperatur an der Oberfläche der von der Heißluft aufgeheizten und zu desinfizierenden Gegenstände ermittelbar, und zwar vorzugsweise kontinuierlich. Im gezeigten Ausführungsbeispiel sind die Infrarotsensoren 15 der Tür 14 zugeordnet, indem sie bevorzugt an der Innenseite der Tür befestigt sind, und zwar so, dass die Infrarotsensoren 15 auf die zu desinfizierenden Gegenstände in der Trommel 11 gerichtet sind, bevorzugt zu unterschiedlichen Stellen. Dadurch wird ein gleichmäßiges Ergebnis der Messung der Oberflächentemperatur der zu desinfizierenden Gegenstände herbeigeführt. Durch die zwei gleichen Infrarotsensoren 15 wird auch eine Redundanz geschaffen, so dass im Falle des Ausfalls eines Infrarotsensors 15 immer noch ein Infrarotsensor 15 die Oberflächentemperatur der zu desinfizierenden Gegenstände im Inneren der drehenden Trommel 11 ermittelt. Es ist denkbar, mehr als zwei vorzugsweise gleiche Infrarotsensoren 15 vorzusehen, gegebenenfalls aber auch nur einen einzigen Infrarotsensor 15. Auch kann der mindestens eine Infrarotsensor 15 an anderen Stellen vorgesehen sein, vorzugsweise an oder in der Trommel. Die Daten der Infrarotsensoren 15 werden dann drahtlos telemetrisch an eine Überwachungseinrichtung der Vorrichtung übertragen.

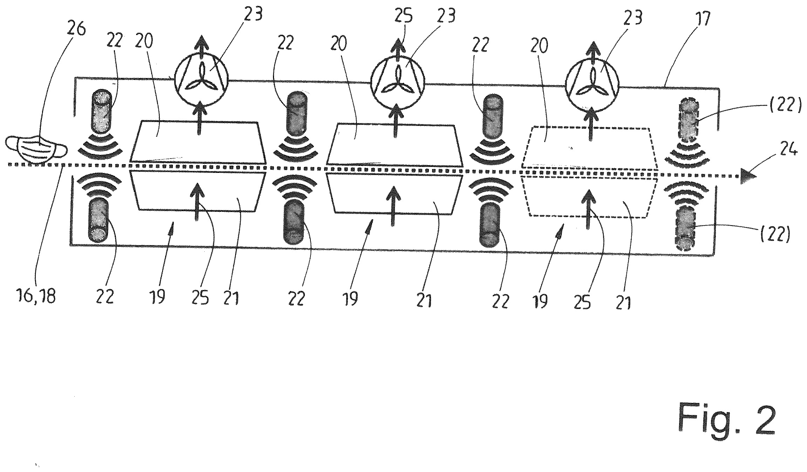

Die in der Fig. 2 gezeigte Vorrichtung arbeitet nach dem Prinzip eines Durchlauftrockners. Dabei werden die zu desinfizierenden Gegenstände, bei denen es sich bevorzugt um empfindliche Gegenstände handelt, wie beispielsweise in der Fig. 2 schematisch dargestellte Schutzmasken 26, die bei der postenweisen Behandlung in einer drehend umlaufend angetriebenen Trommel beschädigt werden könnten.

Bei der Vorrichtung der Fig. 2 werden die zu desinfizierenden Gegenstände aufeinanderfolgend von einem Förderer 16 vorzugsweise kontinuierlich in Durchlaufrichtung 24 durch eine Art Tunnel 17 transportiert, wobei die zu desinfizierenden Gegenstände beim gleichmäßigen Hindurchtransport durch den Tunnel 17 mit Heißluft behandelt und dabei desinfiziert, insbesondere sterilisiert werden.

Beim Förderer 16 handelt es sich um einen Stetigförderer, auf dessen Obertrum 18 die zu desinfizierenden Gegenstände liegen, vorzugsweise einzeln aufeinanderfolgend. Der umlaufend angetriebene Förderstrang des Förderers 16 ist aus einem temperaturbeständigen und luftdurchlässigen Material gebildet. Beispielsweise ist der Förderstrang aus einem oder mehreren perforierten Gurten oder einem Netz bzw. Gitter gebildet. Dadurch ist das Obertrum 18 des Förderers 16 von der zum Desinfizieren der Gegenstände dienenden Heißluft durchströmbar, so dass auch die auf dem Obertrum 18 liegenden Gegenstände von der Heißluft umströmt und gleichmäßig und ausreichend desinfiziert werden.

Bei der gezeigten Vorrichtung sind zur Bildung des Tunnels drei mit geringem Abstand aufeinanderfolgende Hauben 19 vorgesehen. Je nach der gewünschten Länge des Tunnels 17 und/oder der erforderlichen Desinfektionsdauer kann die Anzahl der Hauben 19 kleiner oder vorzugsweise größer als drei sein. Jede der gleichen Hauben 19 ist gebildet aus einem sich mit Abstand über dem Obertrum 18 des Förderers 16 befindenden Hauben-Oberteil 20 und einem unter dem Obertrum 18 angeordneten Hauben-Unterteil 21. Zwischen dem Hauben-Oberteil 20 und dem Hauben-Unterteil 21 jeder Haube 19 läuft das Obertrum 18 mit den darauf liegenden zu desinfizierenden Gegenständen durch die jeweilige Haube 19.

Jede Haube 19 wird von auf die Desinfektions-Temperatur aufgeheizter Heißluft durchströmt, und zwar gemäß dem gezeigten Ausführungsbeispiel der Fig. 2 in Strömungsrichtung 25 vom Hauben-Unterteil 21 zum Hauben-Oberteil 20, also in aufsteigender Richtung. Alternativ kann die Strömungsrichtung umgekehrt sein. Im gezeigten Ausführungsbeispiel wird die aufgeheizte Luft, also Heißluft, dem Hauben-Unterteil 21 zugeführt und nach Durchströmung des Obertrums 18 und Umströmung der zu desinfizierenden Gegenstände aus dem Hauben-Oberteil 20 abgesaugt. Die abgesaugte Luft kann ganz oder teilweise wieder in das Hauben-Unterteil 21 eingeleitet werden. Dadurch findet ein Umluftbetrieb statt. Gegebenenfalls kann auch frische aufgeheizte Luft einen Teil der Umluft ersetzen.

Das Aufheizen der Luft auf die notwendige und/oder gewünschte Desinfektions-Temperatur erfolgt genauso wie bei der Vorrichtung der Fig. 1 beschrieben, worauf Bezug genommen wird. Gleiches gilt für die mindestens eine Einrichtung zum Aufheizen der Luft und/oder die Kontrolle der Desinfektions-Temperatur der Heißluft.

Bei der Vorrichtung der Fig. 2 sind zwischen aufeinanderfolgenden Hauben 19 Sensoren, insbesondere Infrarotsensoren 22, vorgesehen. Ebenso befinden sich jeweils zwei Infrarotsensoren 22 vor der ersten Haube 19 und gegebenenfalls der letzten Haube 19, also am Anfang und am Ende des Tunnels der Vorrichtung. Im gezeigten Ausführungsbeispiel sind jeweils zwei Infrarotsensoren 22 als Paar, vor, zwischen und hinter den Hauben 19 angeordnet, und zwar über und unter dem Obertrum 18. Die Infrarotsensoren 22 sind dabei so gerichtet, dass sie zur Unterseite des Obertrums 18 bzw. zur Oberseite des Obertrums 18 weisen und einander auf einer senkrechten, das Obertrum 18 rechtwinklig schneidenden gemeinsamen Linie bzw. Achse liegen.

Auch die Infrarotsensoren 22 der Vorrichtung der Fig. 2 dienen dazu, die Oberflächentemperatur der durch die Vorrichtung vom Förderer 16 im Durchlaufprinzip kontinuierlich hindurchtransportierten zu desinfizierenden Gegenstände laufend zu messen. Es sind so die Oberflächentemperaturen der Gegenstände vor der ersten Haube 19, zwischen den Hauben 19 und optional, das heißt nicht zwingend, der letzten Haube 19 ermittelbar, und zwar vorzugsweise fortlaufend. Dadurch ist eine laufende Kontrolle des Desinfektionsvorgangs beim Hindurchtransportieren der zu desinfizierenden Gegenstände durch die nach dem Durchlaufprinzip arbeitende Vorrichtung gewährleistet.

Bei der Vorrichtung der Fig. 2 erstreckt sich der Tunnel 19 über die gesamte Länge der Desinfektionsstrecke, also zumindest im Bereich aller Hauben 19 und/oder aller Infrarotsensoren 22. Der Tunnel 17 bildet eine Einhausung, die die Abstrahlung von der Wärmeenergie nach außen reduziert, insbesondere wenn die Einhausung zusätzlich isoliert ist. Es wird so auch verhindert, dass in den Abschnitten zwischen aufeinanderfolgenden Hauben 19 sowie vor und hinter der Haube, wo sich die Infrarotsensoren 22 befinden, keine Wärme nach außen abstrahlen und dadurch verloren gehen kann.

In der Fig. 2 sind jeder Haube 19, insbesondere über jedem Hauben-Oberteil 20 angeordnete Ventilatoren 23 dargestellt. Diese dienen zur Erzeugung einer HeißluftStrömung durch die jeweilige Haube 19, insbesondere einer Umluftströmung durch die betreffende Haube 19.

Die mikrobiell kontaminierten Gegenstände werden gemäß dem erfindungsgemäßen Verfahren trocken und/oder im trockenen Zustand über einen bestimmten ununterbrochenen Zeitraum hinweg mit Heißluft, die die erforderliche Desinfektions-Temperatur aufweist, behandelt. Dabei werden die zu desinfizierenden Gegenstände einer Heißluftströmung ausgesetzt und soweit desinfiziert, dass ihre biologische Kontamination beseitigt wird, insbesondere Keime, Bakterien, Viren, Sporen, Pilze oder dergleichen vollständig oder zumindest nahezu vollständig abgetötet werden. Das hat eine Sterilisation der kontaminierten Gegenstände zur Folge, so dass diese nicht mehr infektiös sind.

Die Temperatur der Heißluft, also die Desinfektions-Temperatur, ist so gewählt, dass sie geringfügig über der Temperatur liegt, der die zu desinfizierenden Gegenstände schadlos wiederstehen können. Vorzugsweise liegt die Desinfektions-Temperatur 5% bis 20% unter der Temperatur, womit die Gegenstände maximal behandelt werden können, ohne Schaden zu nehmen. Diese Temperatur liegt vorzugsweise im Bereich von 70°C bis 98°C, insbesondere 85°C bis 95°C.

Die zu desinfizierenden Gegenstände werden bevorzugt 5 min bis 15 min, insbesondere 8 min bis 12 min, vorzugsweise ununterbrochen mit Heißluft behandelt, wobei in dieser Zeit die Heißluft stets mindestens die Desinfektions-Temperatur aufweisen muss.

In der Vorrichtung der Fig. 1 werden die zu desinfizierenden Gegenstände postenweise, also in einer größeren Anzahl, gleicher oder unterschiedlicher, jedoch gleichwohl zu desinfizierender Gegenstände Heißluft, insbesondere einer Heißluftströmung, ausgesetzt. Die Gegenstände werden dazu in der drehend angetriebenen Trommel 11 während der vorgesehenen Behandlungsdauer, die der Desinfektionsdauer entspricht, in einer Heißluftströmung mit Heißluft desinfiziert, die während der gesamten Behandlungsdauer eine Temperatur von mindestens der Desinfektions-Temperatur aufweist.

Durch den Lufterhitzern zugeordnete Temperatursensoren und/oder insbesondere den mindestens einen Infrarotsensor 15, vorzugsweise mindestens zwei Infrarotsensoren 15, wird während der gesamten Behandlungsdauer, nämlich Desinfektionsdauer, die Temperatur der Heißluft bemessen. Von den vorzugsweise mehreren Infrarotsensoren 15 wird vor allem die Oberflächentemperatur der zu desinfizierenden Gegenstände fortlaufend gemessen.

Wird von den vorzugswiese mehreren Infrarotsensoren 15 festgestellt, dass die Temperatur an der Oberfläche der zu desinfizierenden Gegenstände die vorgegebene Desinfektions-Temperatur unterschreitet, wird die Erhitzung der Heißluft so gesteuert oder geregelt, dass diese stärker aufgeheizt wird, bis der mindestens eine Infrarotsensor 15 wieder eine Oberflächentemperatur der Gegenstände feststellt, die der vorgegebenen Desinfektions-Temperatur entspricht.

Die Zeitdauer, in der eine Oberflächentemperatur der Gegenstände unter der vorgegebenen Desinfektions-Temperatur von mindestens einem Infrarotsensor 15 festgestellt wurde, wird nicht als Desinfektionszeit gezählt. Auf diese Weise ist sichergestellt, dass über den gesamten vorgegebenen Zeitraum, nämlich die Desinfektionszeit, die Temperatur der Heißluft und/oder die Oberflächentemperatur der zu desinfizierenden Gegenstände stets der Desinfektions-Temperatur entspricht oder innerhalb einer vorgegebenen Toleranz über der Desinfektions-Temperatur liegt. Dadurch wird sichergestellt, dass die Gegenstände über den vorgegebenen Zeitraum hinweg mit Heißluft behandelt werden, die mindestens der vorgegebenen Desinfektions-Temperatur entspricht und/oder an der Oberfläche der zu desinfizierenden Gegenstände über den vorgegebenen Zeitraum hinweg eine der Desinfektions-Temperatur mindestens entsprechende Temperatur gemessen wird.

Nach Ablauf der vorgegebenen Zeitdauer werden die desinfizierten Gegenstände postenweise aus der Trommel 11 entladen, wonach der nächste Posten zu desinfizierender Gegenstände in die Trommel 11 geladen werden kann. Sofern es sich hierbei um andere Gegenstände handelt, werden diese mit einer anderen Desinfektions-Temperatur und/oder über einen anderen Zeitraum hinweg desinfiziert.

Mit der Vorrichtung der Fig. 2 werden die zu desinfizierenden Gegenstände einzeln aufeinanderfolgend desinfiziert. Dabei werden vom Förderer 16 die zu desinfizierenden Gegenstände im Durchlaufverfahren in Durchlaufrichtung 24 durch den Tunnel 17, nämlich alle aufeinanderfolgenden Hauben 19, der Vorrichtung hindurchtransportiert, wobei die zu desinfizierenden Gegenstände während der gesamten Desinfektionsdauer auf dem luftdurchlässigen Obertrum 18 des Förderers 16 ruhen. Die Transportgeschwindigkeit des Förderers 16 und die Länge des Tunnels 17 sind so aufeinander abgestimmt, dass beim kompletten Durchlauf des jeweiligen zu desinfizierenden Gegenstands durch den Tunnel 17 dieser textile Gegenstand die vorgesehene Zeitdauer sich im Tunnel 17 befindet und dabei mit der vorgegebenen Desinfektions-Temperatur behandelt, insbesondere umströmt wird. Die Behandlungsdauer und die Desinfektions-Temperatur entsprechen bei der Desinfektion mit der Vorrichtung der Fig. 2 den Werten der Desinfektion mit der Vorrichtung der Fig. 1.

Die Desinfektionsdauer beginnt zu laufen, wenn die Infrarotsensoren 22 vor der ersten Haube 19 eine ausreichende Desinfektions-Temperatur festgestellt haben. Wenn sich diese Desinfektions-Temperatur während der gesamten Behandlungsdauer nicht ändert oder innerhalb zusätzlicher Toleranzen bleibt, erfolgt die Desinfektion ununterbrochen durchgehend bis zum Ablauf der vorgesehenen Desinfektionsdauer. Wenn jedoch irgendwelche Infrarotsensoren 22 feststellen, dass irgendwo im Bereich der Behandlungsstrecke vor und/oder zwischen den Hauben 19 die Desinfektions-Temperatur unter die Vorgaben sinkt, wird die Messung der Desinfektionszeit angehalten. Die Messung beginnt erst dann wieder, wenn insbesondere die Infrarotsensoren 22, vorzugsweise alle Infrarotsensoren 22, festgestellt haben, dass die Desinfektions-Temperatur wieder im Bereich der vorgesehenen Desinfektions-Temperatur liegt. Dann wird die Messung der Behandlungsdauer wieder gestartet und die Behandlungsdauer beendet, wenn die vorgesehene Behandlungsdauer mit vorgegebener Desinfektions-Temperatur erreicht ist. Idealerweise sollte zu diesem Zeitpunkt der betreffende Gegenstand sich am Ende des Tunnels 17, also am Auslauf der letzten Haube 19 befinden.

Die Transportgeschwindigkeit des Förderers 16 wird bezogen auf die festgelegte Länge des Tunnels 17 so berechnet, dass der betreffende zu desinfizierende Gegenstand nach Ablauf der Desinfektionsdauer durch den Tunnel 17 hindurchgelaufen ist, insbesondere sich am Ende des Tunnels 17 befindet. Falls festgestellt wird, dass die Desinfektions-Temperatur unter den vorgegebenen Wert gesunken ist, wird der Förderer 16 gestoppt oder seine Transportgeschwindigkeit verringert. In Abhängigkeit von der Zeitdauer bis zur Wiederherstellung von Heißluft mit der vorgesehenen Desinfektions-Temperatur wird danach die Transportgeschwindigkeit des Förderers 16 so angepasst, dass am Ende des Tunnels 17 der betreffende Gegenstand sich über den vorgegebenen Zeitraum hinweg bei ausreichender Desinfektions-Temperatur im Tunnel 17 befunden hat. Dadurch wird gewährleistet, dass auch bei einer Unterbrechung der Desinfektionsbehandlung aufgrund nicht ausreichend hoher Desinfektions-Temperatur der jeweils zu desinfizierende Gegenstand ausreichend lange der vorgesehenen Desinfektions-Temperatur ausgesetzt ist und dadurch eine zuverlässige Beseitigung von Keimen, Viren, Bakterien und sonstigen biologischen Kontaminationen des jeweiligen Gegenstands bis hin zur Sterilisation desselben gewährleistet ist und somit von den desinfizierten Gegenstand keine Infektionsgefahr mehr ausgeht.

Das erfindungsgemäße Verfahren lässt sich nicht nur mit den beiden zuvor beschriebenen Vorrichtungen durchführen, sondern auch mit Vorrichtungen anderer Art, anderer Arbeitsweise und/oder anderem Aufbau.

### Bezugszeichenliste:

- 10: Gehäuse
- 11: Trommel
- 12: Vorderwand
- 13: Öffnung
- 14: Tür
- 15: Infrarotsensor
- 16: Förderer
- 17: Tunnel
- 18: Obertrum
- 19: Haube
- 20: Hauben-Oberteil
- 21: Hauben-Unterteil
- 22: Infrarotsensor
- 23: Ventilator
- 24: Durchlaufrichtung
- 25: Strömungsrichtung
- 26: Schutzmaske

## Patentansprüche

1. Verfahren zum Desinfizieren von kontaminierten Gegenständen, vorzugsweise biologisch kontaminierten textilen Gegenständen, wobei die kontaminierten Gegenstände im trockenen Zustand mit Heißluft beaufschlagt werden.

2. Verfahren nach Anspruch 1, **dadurch gekennzeichnet, dass** die zu desinfizierende Gegenstände von einer Vorrichtung nach Art eines Trockners mit Heißluft beaufschlagt werden.

3. Verfahren nach einem der vorhergehenden Ansprüche, **dadurch gekennzeichnet, dass** die trockenen biologisch kontaminierten Gegenstände von der Heißluft durchströmt werden, vorzugsweise mit umgewälzter Heißluft und/oder heißer Umluft.

4. Verfahren nach einem der vorhergehenden Ansprüche, **dadurch gekennzeichnet, dass** die Heißluft auf eine Desinfektions-Temperatur aufgeheizt wird, die 5% bis 20% unter derjenigen Temperatur liegt, womit die jeweiligen Gegenstände maximal beschädigungslos beaufschlagbar sind und/oder zum sicheren Abtöten biologischer Kontaminationen, wie Keime, Bakterien, Viren, Pilze oder dergleichen, die Desinfektions-Temperatur im Bereich von 70°C bis 98°C liegt.

5. Verfahren nach einem der vorhergehenden Ansprüche, **dadurch gekennzeichnet, dass** die kontaminierten Gegenstände über einen bestimmten Zeitraum hinweg, vorzugsweise einen bestimmten ununterbrochenen Zeitraum hinweg, mit der mindestens Desinfektions-Temperatur aufweisenden Heißluft beaufschlagt werden, insbesondere der Zeitraum der Beaufschlagung der kontaminierten Gegenstände mit der Desinfektions-Temperatur aufweisenden Heißluft 5 min bis 15 min, vorzugsweise 5 min bis 12min, beträgt.

6. Verfahren nach einem der vorhergehenden Ansprüche, **dadurch gekennzeichnet, dass** die Desinfektions-Temperatur während der Beaufschlagung der kontaminierten Gegenstände mit der Heißluft, vorzugsweise fortlaufend, mit mindestens einem Messorgan, insbesondere mit mehreren Messorganen, gemessen wird.

7. Verfahren nach Anspruch 6, **dadurch gekennzeichnet, dass** vom mindestens einen Messorgan die Oberflächentemperatur der zu desinfizierenden Gegenstände berührungslos gemessen wird.

8. Verfahren nach einem der vorhergehenden Ansprüche, **dadurch gekennzeichnet, dass** nass zu behandelnde textile Gegenstände vor der Nassbehandlung im noch trockenen Zustand durch Beaufschlagung mit Heißluft desinfiziert werden.

9. Verfahren nach einem der vorhergehenden Ansprüche, **dadurch gekennzeichnet, dass** die zu desinfizierenden Gegenstände postenweise, vorzugsweise als Posten sortierter zu desinfizierender Gegenstände, in einer drehend antreibbaren und für die Heißluft durchgängigen und/oder durchströmbaren Trommel (11) mit Heißluft beaufschlagt werden.

10. Verfahren nach einem der Ansprüche 1 bis 8, **dadurch gekennzeichnet, dass** die zu desinfizierenden Gegenstände im Durchlauf durch einen Tunnel (17) mit der Heißluft, vorzugsweise den Tunnel (17) durchströmenden Heißluft, beaufschlagt werden, wobei während dieser Beaufschlagung mit Heißluft die zu kontaminierenden Gegenstände nacheinander, insbesondere einzeln, von einem Förderer (16) durch den Tunnel (17) transportiert werden.

11. Verfahren nach einem der vorhergehenden Ansprüche, **dadurch gekennzeichnet, dass** als Heißluft aufgeheizte Luft, insbesondere trockene, aufgeheizte Luft, verwendet wird.
